Europäisches Patentamt

European Patent Office

Office européen des brevets

(11) Numéro de publication: **0 165 157**
**A1**

## (12) DEMANDE DE BREVET EUROPEEN

(21) Numéro de dépôt: **85401033.7**

(22) Date de dépôt: **28.05.85**

(51) Int. Cl.⁴: **A 61 B 6/00**
**A 61 B 6/04**

(30) Priorité: **30.05.84 FR 8408488**

(43) Date de publication de la demande:
**18.12.85 Bulletin 85/51**

(84) Etats contractants désignés:
**DE IT NL**

(71) Demandeur: **THOMSON-CGR**
**13, square Max-Hymans**
**F-75015 Paris(FR)**

(72) Inventeur: **Le Sonn, Marcel**
**THOMSON-CSF SCPI 173, bld Haussmann**
**F-75379 Paris Cedex 08(FR)**

(74) Mandataire: **Grynwald, Albert et al,**
**THOMSON-CSF SCPI 173, Bld Haussmann**
**F-75379 Paris Cedex 08(FR)**

(54) **Dispositif radiologique à accès total au patient.**

㊗ La présente invention concerne un dispositif radiologique à accès total au patient, au type comportant un émetteur (9) et an récepteur (10) de rayonnement X, un panneau porte-patient (8), organisés de manière à former un ensemble basculant autour d'un axe horizontel (4).

Le panneau porte-patient (8) est solidarisé à un support principal (7), par l'intermédiaire d'un unique bras support (31), de manière à ménager, entre ledit panneau port-patient (8) et un côté (32) dudit support principal (7), un espace (D) suffisant pour le passage d'un praticien.

./...

# DISPOSITIF RADIOLOGIQUE A ACCES TOTAL AU PATIENT

La présente invention concerne un dispositif radiologique à accès total au patient, destiné notamment à une utilisation dans le cadre du radiodiagnostic ; elle concerne particulièrement les dispositifs du type comportant un système émetteur et récepteur de rayonnement X et un panneau porte-patient, formant un ensemble capable de basculer autour d'un axe horizontal.

L'examen radiologique d'un patient nécessite fréquemment qu'un opérateur, ou un médecin, intervienne près du patient ; cette intervention exigeant l'accès à l'un ou l'autre des côtés longitudinaux du panneau porte-patient.

Cet accès est relativement aisé dans le cas d'installations formées par une table d'examen, fixée au sol indépendemment d'une source et d'un récepteur de rayonnement X, ces derniers étant par exemple supportés à l'aide d'un statif fixé au plafond. Par contre, l'accès à au moins un côté longitudinal du panneau porte-patient est rendu impossible, avec l'agencement actuel de dispositif radiologique du type basculant, où, le panneau porte-patient, la source et le récepteur de rayonnement X sont intégrés dans un ensemble capable de basculer en conservant à chacun de ces éléments leur position relative. Dans de tels dispositifs, la source de rayonnement X et le récepteur qui lui est associé, sont généralement supportés aux extrémités opposés d'un même bras, en forme d'arceau, lui-même mobile d'une part autour d'un axe horizontal, et d'autre part dans un plan transversal à celui du panneau porte-patient, de manière à permettre des examens sous des incidences complexes en conservant un même point isocentre.

Aussi, les moyens mécaniques nécessaires à ces déplacements ainsi qu'au basculement du dispositif radiologique, compte tenu de l'importance des masses en mouvement, occupent sur un côté longitudinal du panneau porte-patient un espace considérable, qui empêche l'accès à ce côté.

La présente invention concerne un dispositif radiologique à accès total au patient, du type basculant autour d'un axe horizontal, dans lequel l'accès total au patient est aménagé grâce à une nouvelle disposition du panneau porte-patient, par rapport aux autres éléments du dispositif radiologique de l'invention.

Selon l'invention, un dispositif radiologique à accès total au patient, du type basculant autour d'un axe horizontal, comportant un socle fixe, un support basculant monté en rotation autour d'un axe horizontal par rapport audit socle fixe, un support principal solidaire dudit support basculant et comportant un axe longitudinal perpendiculaire audit axe horizontal de basculement, ledit support principal supportant un panneau porte-patient ayant une longueur parallèle audit axe longitudinal, ledit support principal supportant en outre, par l'intermédiaire d'un support d'arceau, un arceau muni d'une source et d'un récepteur de rayonnement X, est caractérisé en ce que ledit panneau porte-patient est supporté par un unique bras support solidarisé audit panneau porte-patient et audit support principal selon un axe sensiblement perpendiculaire audit axe longitudinal, de manière à déporter latéralement ledit panneau porte-patient par rapport audit support principal et à constituer, entre un côté dudit support principal et un bord longitudinal intérieur dudit panneau porte-patient, une distance suffisante au passage d'un praticien.

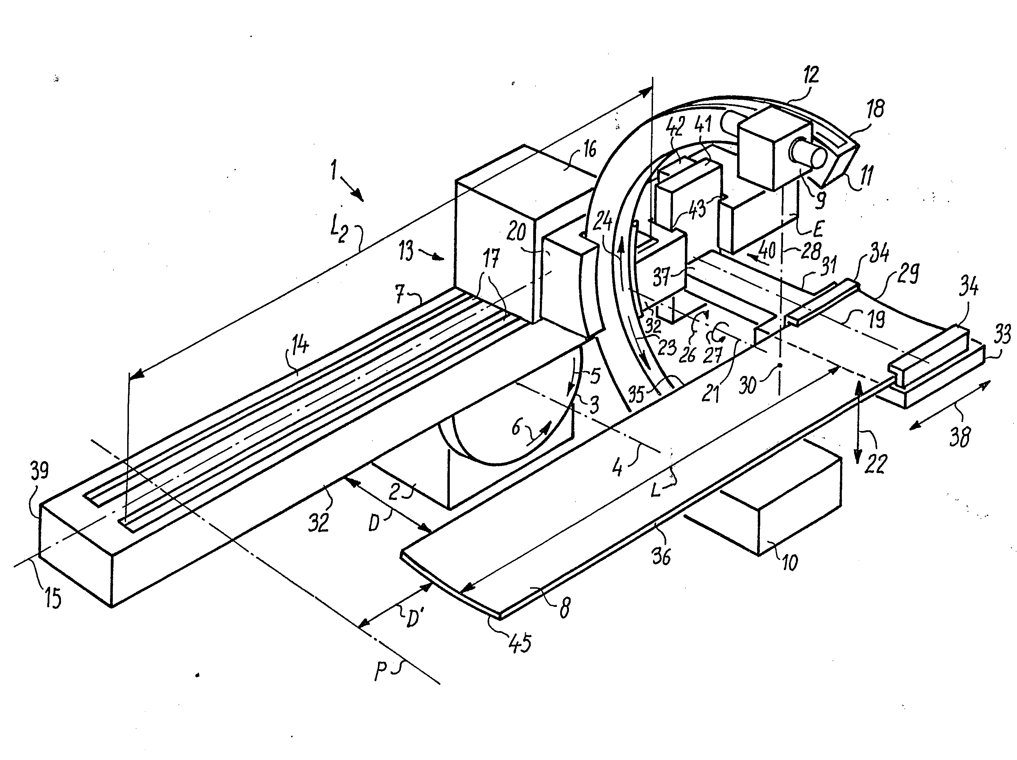

L'invention sera mieux comprise à l'aide de la description qui va suivre, donnée à titre d'exemple non limitatif, et faite en référence à la figure unique jointe.

La figure représente, par une vue en perspective, un dispositif radiologique conforme à l'invention.

La figure montre un dispositif radiologique 1 selon l'invention, du type basculant. Il comporte un socle 2, fixe, sur lequel est monté, par des moyens classiques non représentés, un support basculant 3 capable d'un mouvement de rotation sur lui-même autour d'un premier axe horizontal 4, dans un premier ou un second sens montré par les première et seconde flèches 5,6 ; cet axe horizontal 4 constituant l'axe de basculement du dispositif radiologique 1.

Un support principal 7, comportant un axe longitudinal 15, est disposé au-dessus du support basculant 3 auquel il est solidarisé par des moyens traditionnels (non représentés), de manière que son axe longitudinal 15 soit perpendiculaire au premier axe horizontal 4, et soit contenu dans un plan parallèle au plan dans lequel s'effectue la rotation du support basculant 3. Le support principal 7 peut ainsi basculer autour du premier axe horizontal 4, et entraîne dans ce mouvement des moyens qu'il supporte, ces moyens étant essentiellement, dans l'exemple non limitatif décrit, un panneau porte-patient 8, une source 9 de rayonnement X et un récepteur 10 de ce rayonnement X.

La source 9 et le récepteur 10 de rayonnement X sont portés de manière classique, chacun à des extrémités 11 opposées d'un bras 12, constitué dans l'exemple non limitatif décrit, selon la forme d'un arceau. Le bras ou arceau 12 est supporté par le support principal 7, par l'intermédiaire d'un support d'arceau 13, comportant d'une part un chariot 16, mobile parallèlement à l'axe longitudinal 15 du support principal 7 ; le déplacement du chariot 16 s'effectuant par des moyens classiques tels que par exemple, des rails 17 encastrés dans une face supérieure 14 du support principal 7, et coopérant avec des moyens de roulement (non représentés) dont est muni le chariot 16. Le support d'arceau 13 comporte d'autre part un support intermédiaire 20 dans lequel est engagé l'arceau 12 ; le support intermédiaire 20 est monté sur le chariot 16 en rotation par rapport à ce dernier autour d'un second axe horizontal 21. Le support intermédiaire 20 comporte en outre, d'une manière classique non représentée, des moyens de déplacement tels qu'une poulie crantée par exemple coopérant avec une crémaillère 18 disposée le long de l'arceau 12, de manière à permettre un déplacement circulaire de l'arceau dans son plan, dans l'un ou l'autre des sens montrés par des troisième et quatrième flèches 23, 24.

La rotation du support intermédiaire 20 autour du second axe horizontal 21, telle que montré par les cinquième et sixième flèches 26, 27, permet de basculer le plan de l'arceau autour de ce second axe horizontal 21, de manière à réaliser des incidences longitudinales, et le déplacement circulaire de l'arceau 12 dans son plan comme montré par les troisième et quatrième flèches 23, 24 permet de réaliser des incidences transversales (non représentées). La combinaison de ces incidences permet de réaliser des incidences complexes, dans lesquelles un axe de travail 28 établi entre la source et le récepteur 9, 10, passe toujours par un point isocentre 30 ; ces conditions pouvant être reproduites tout le long d'une longueur utile L du panneau porte-patient 8, grâce au déplacement du chariot 16 parallèlement à l'axe longitudinal 15.

Il est à noter que les mouvements précédemment décrits, du support basculant 3, du chariot 16, du support intermédiaire 20 et de l'arceau 12, peuvent être motorisés grâce à des moyens moteurs classiques, non représentés pour plus de clarté de la description.

Le panneau porte-patient 8 est supporté par un unique bras support 31. Dans l'exemple non limitatif décrit, le bras support 31 est disposé selon un axe 19 sensiblement perpendiculaire à l'axe longitudinal 15 du support principal 7, et au panneau porte-patient 8, la longueur utile L de ce dernier étant sensiblement parallèle à l'axe longitudinal 15. Le panneau porte-patient 8 est ainsi supporté parallèlement au support principal 7, par rapport auquel il est déporté latérallement, le long d'un côté intérieur 32 de ce dernier et à une distance D qui représente la longueur utile du bras support 31 ; cette distance D permettant le passage d'un praticien entre le support principal 7 et le panneau porte-patient 8.

Le bras support 31 comporte une première extrémité 37 par laquelle il est solidarisé au support principal 7, et une seconde extrémité 33 par laquelle il est solidarisé au panneau porte-patient 8. La seconde extrémité 33 peut être solidarisée au panneau porte-patient 8, par des moyens classiques tels que des vis (non représentées), ou ainsi que dans l'exemple non limitatif décrit, par des glissières 34 dont est munie la seconde extrémité 33 du bras support 31 ; ces glissières 34 maintenant le panneau porte-patient 8 par ses bords longitudinaux intérieur et extérieur respectivement repérés 35, 36. Il est alors possible de déplacer le panneau porte-patient 8 selon sa longueur L, par rapport au bras support 31 comme montré par la septième flèche 38.

Cette manière de supporter le panneau porte-patient 8 permet un accès total au patient (non représenté), et notamment l'accès à au bord longitudinal intérieur 35 du panneau porte-patient 8, ce bord étant inaccessible dans un dispositif radiologique de type basculant selon l'art antérieur.

Dans le dispositif radiologique 1 selon l'invention tel que montré dans l'exemple non limitatif de la figure, le panneau porte-patient 8 est solidarisé par sa première extrémité 29 au bras support 31, étant ainsi supporté entièrement en porte-à-faux ; il est alors possible de positionner l'arceau 12 à proximité du bras support 31 grâce au déplacement du chariot 16, et de libérer le long du panneau porte-patient 8 l'espace, représenté par la distance D, entre son bord longitudinal intérieur 35 et le bord intérieur 32 du support principal 7. Il est également possible dans cette configuration, d'accéder au bord longitudinal intérieur 35 du panneau porte-patient 8, en positionnant l'arceau 12 vers une extrémité 39 du support principal 7, à une position symbolisée sur la figure par une ligne en traits pointillés repérée P, de manière à ménager entre cette position P de l'arceau 12 et une seconde extrémité 45 du panneau porte-patient 8, une distance D' permettant le passage d'un praticien ; l'espace entre le bord intérieur 32 du support principal 7 et le bord longitudinal intérieur 35 du panneau porte-patient 8 étant également libéré. Ceci est obtenu dans l'exemple non limitatif décrit, en conférant au chemin de déplacement constitué par les rails 17 parallèles à l'axe longitudinal 15, une longueur $L_2$ supérieure à la longueur utile L du panneau porte-patient 8 ; il est à remarquer que la longueur $L_2$ du chemin de déplacement 17 peut être diminuée en procédant à un déplacement, selon la septième flèche 38, du panneau porte-patient 8.

Un autre avantage de la position déportée latéralement, du panneau porte-patient 8 par rapport au support principal 7, réside en ce que l'espace situé sous le panneau porte-patient 8 est considérablement libéré, par rapport à un ensemble radiologique selon l'art antérieur ; ceci permet un second déplacement du panneau porte-patient 8, perpendiculairement à son plan comme montré par une huitième flèche 22, avec une amplitude beaucoup plus importante que dans l'art antérieur. Cette dernière fonction est obtenue grâce à un dispositif d'ascenseur 40 dont est muni le support principal 7, et par l'intermédiaire duquel le bras support 31 est solidarisé au support principal 7. Dans l'exemple non limitatif décrit, le dispositif 40 comporte une potence 41, munie d'une barre à crémaillère 42 lui permettant de coopérer avec des moyens moteurs classiques (non représentés), de manière à coulisser dans des gorges 43 pratiquées dans l'épaisseur E du support principal 7 ; la première extrémité 37 du bras support 31 étant fixée, par des moyens traditionnels non représentés, à la potence 41.

Cette description constitue un exemple non limitatif du dispositif radiologique 1 conforme à l'invention, l'essentiel étant que ce dispositif radiologique comporte un panneau porte-patient 8, formant avec au moins une source 9 et un récepteur 10 de rayonnement X, un ensemble basculant porté par un support principal 7, et que le panneau porte-patient 8 soit supporté par un unique bras support 31 dans une position déportée par rapport à ce support principal 7, le long d'un côté de ce dernier.

REVENDICATIONS

1. Dispositif radiologique à accès total au patient, du type basculant autour d'un axe horizontal (4), comportant un socle (2) fixe, un support basculant (3) monté en rotation autour dudit axe horizontal (4) par rapport audit socle (2), un support principal (7) solidaire dudit support basculant (3) et comportant un axe longitudinal (15) perpendiculaire audit axe horizontal (4) de basculement, ledit support principal (7) supportant un panneau porte-patient (8) ayant une longueur (L) parallèle audit axe longitudinal (15), ledit support principal (7) supportant en outre, par l'intermédiaire d'un support d'arceau (13), un arceau (12) muni d'une source et d'un récepteur (9, 10) de rayonnement X, caractérisé en ce que ledit panneau porte-patient (8) est supporté par un unique bras support (31) solidarisé audit panneau porte-patient (8) et audit support principal (7) selon un axe sensiblement perpendiculaire audit axe longitudinal (15), de manière à déporter latéralement ledit panneau porte-patient (8) par rapport audit support principal (7) et à constituer, entre un côté (32) dudit support principal (7) et un bord (35) dudit panneau porte-patient (8), une distance (D) suffisante au passage d'un praticien.

2. Dispositif radiologique selon la revendication 1, caractérisé en ce que ledit bras support (31) est fixé à une extrémité (29) du panneau porte-patient (8), ledit panneau porte-patient (8) étant entièrement supporté en porte-à-faux.

3. Dispositif radiologique selon la revendication 1, caractérisé en ce que ledit bras support (31) comporte des glissières (34) permettant un déplacement (38) dudit panneau porte-patient (8) selon sa longueur (L).

4. Dispositif radiologique selon l'une des revendications précédentes, caractérisé en ce que le support principal (7) comporte un chemin de déplacement (17), parallèle audit axe longitudinal (15) et

8

permettant le déplacement du support d'arceau (13), ledit chemin de déplacement comportant une longueur ($L_2$) supérieure à la longueur (L) dudit panneau porte-patient (8).

5. Dispositif radiologique selon l'une des revendications précédentes, caractérisé en ce qu'il comporte un dispositif d'ascenseur (40), par l'intermédiaire duquel ledit bras support (31) est solidarisé audit support principal (7), permettant un déplacement (22) dudit panneau porte-patient (8) perpendiculairement à son plan.

0165157

Office européen
des brevets

**RAPPORT DE RECHERCHE EUROPEENNE**

EP 85 40 1033

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int. Cl 4) |
|---|---|---|---|
| A | US-A-3 500 045 (G. ROSSI) <br> * En entier * | 1-5 | A 61 B 6/00 <br> A 61 B 6/04 |
| | --- | | |
| A | DE-A-2 519 242 (K. SCHWARZER) <br> * Page 3, ligne 20 - page 5, ligne 26; figures 1-4 * | 1-4 | |
| | --- | | |
| A | DE-A-2 046 207 (J.W. PEGRUM et al.) <br> * Page 6, ligne 5 - page 8, ligne 24; figures 1,2 * | 1-5 | |
| | --- | | |
| A | FR-A-2 342 704 (SIEMENS AG) <br> * Page 3, ligne 13 - page 4, ligne 21; figure 1 * | 1-4 | |
| | --- | | DOMAINES TECHNIQUES RECHERCHES (Int. Cl.4) |
| A | DE-B-1 029 527 (F. HOFMANN GmbH) <br> * En entier * | 1-5 | A 61 B |
| | ----- | | |

Le présent rapport de recherche a été établi pour toutes les revendications

| Lieu de la recherche | Date d'achèvement de la recherche | Examinateur |
|---|---|---|
| LA HAYE | 29-08-1985 | RIEB K.D. |